# EUROPEAN PATENT APPLICATION

(11) **EP 4 838 534 A1**
(43) Date of publication of application: **11.11.2026**
(21) Application number: 25174941.2
(22) Date of filing: 08.05.2025
(51) Int. Cl.: G16C 60/00, G16C 20/30, G16C 20/70

(54) **A METHOD FOR DETERMINING ADDITONAL PREDICTED TEST BEHAVIOUR CHARACTERISTICS OF A VIRTUAL MATERIAL**

(71) Applicant: Henkel AG & Co. KGaA, 40589 Düsseldorf (DE)
(72) Inventor: Manickaguptha, Adhiseshan, 40599 Düsseldorf (DE); Sadeghi, Mohammad Zamaan, 40591 Düsseldorf (DE); Lammerschop, Olaf, 47802 Krefeld (DE)

(57) **Abstract**

The disclosure relates to a method for determining additional predicted test behavior characteristics of a virtual material, wherein the virtual material is associated with predicted test behavior characteristics with respect to at least one virtual material test, the method performed by a processor, the method comprising: applying the virtual material and the associated predicted test behavior characteristics with respect to at least one virtual material test to a machine-learning model, wherein the machine-learning model is adapted to a plurality of training data sets, and determining the additional predicted test behavior characteristics of the virtual material using the machine-learning model.

## Description

### Technical Field

Various embodiments relate generally to a method for determining additional predicted test behavior characteristics of a virtual material.

### Background

WO 2022/019971 A1 discloses a system and a method that receive first input from a user of a candidate formulation recipe, and second input from the user of target properties and target property constraints. The system inputs the first input into a machine learning model, the model having been trained using historical training data, each element of the historical training data corresponding to a known formulation having a known feature representation, each known formulation having associated properties and statistical representations of each feature of the known formulation that form the known feature representation. The system receives as output from the model a predicted property of a candidate formulation derived using the first input and the likelihood that the candidate formulation satisfies the target property constraints using the second input. The system generates a predicted likelihood that the predicted property satisfies the second input for display to the user.

KR 20210154581 A discloses a method for predicting formulation of a structural epoxy adhesive using machine learning, and more particularly, a method for predicting formulation of a structural epoxy adhesive using artificial neural networks that are one of the machine learning methods. Various correlations appearing between the above independent variables can be established to predict lap shear strength of the epoxy adhesive and low temperature impact peel strength of -30 °C. to -50 °C.

Adhesives are formulated by the chemist based on the requirements from the customers. Once the requirements are defined, the chemist starts mixing different combination of materials to satisfy the defined requirement. Attaining the desired formulation for the requirement involves several iterations, which is a tedious and challenging process. Once the desired formulation is finalized, several tests are carried out and adhesives are tested in customer simulation models numerically to check if the adhesives can pass the requirement criteria. Modelling the numerical behaviour of an adhesive and performing finite element simulation or computational fluid dynamics simulation is a complex process. Characterizing the adhesive behaviour for numerical simulation requires several experiments such as dogbone, lapshear test and etc. for structural performance, thermal conductivity and specific heat test for thermal performance and capillary tube and parallel plate test for rheological performance. This process of formulating the material iteratively and then characterizing the adhesive behaviour using complex experimental efforts is referred as a "classical approach" in this disclosure. This "classical approach" is tedious, time consuming and requires a lot of physical effort.

### Summary

This disclosure provides a predictive machine learning method to identify the structural, thermal and rheological behavior of an adhesive using focused test and the database of simulation results. A quick and faster process to address the customer need is provided.

The disclosure relates to a method for determining additional predicted test behavior characteristics of a virtual material. The virtual material is associated with predicted test behavior characteristics with respect to at least one virtual material test. The method is performed by a processor. The method includes applying the virtual material and the associated predicted test behavior characteristics with respect to at least one virtual material test to a machine-learning model. The machine-learning model is adapted to a plurality of training data sets. Each training data set of the plurality of training data sets includes a virtual reference material and associated simulated test behavior characteristics for a plurality of virtual reference material tests referring to the respective virtual reference material. The virtual reference materials are different from the virtual material and at least some virtual reference material tests are different from the at least one virtual material test. By using the machine-learning model additional predicted test behavior characteristics of the virtual material are determined.

### Brief Description of the Drawings

In the drawings, like reference characters generally refer to the same parts throughout the different views. The drawings are not necessarily to scale, emphasis instead generally being placed upon illustrating the principles of the invention. In the following description, various embodiments of the invention are described with reference to the following drawings, in which:
FIG. 1 shows a device for performing the method according to an exemplary embodiment of the disclosure;
FIG. 2 shows input and output data for several components of the device for performing the method according to an exemplary embodiment of the disclosure;
FIG. 3 shows three steps of the method according to an exemplary embodiment of the disclosure;
FIG: 4 shows more steps of the method according to an exemplary embodiment of the disclosure; and
FIG. 5 shows incorporating the determination of the virtual material into the method according to an exemplary embodiment of the disclosure.
FIG. 6 shows the determination of the virtual material in more detail according to an exemplary embodiment of the disclosure.

### Description

The following detailed description refers to the accompanying drawings that show, by way of illustration, specific details and embodiments in which the invention may be practiced.

The word "exemplary" is used herein to mean "serving as an example, instance, or illustration". Any embodiment or design described herein as "exemplary" is not necessarily to be construed as preferred or advantageous over other embodiments or designs.

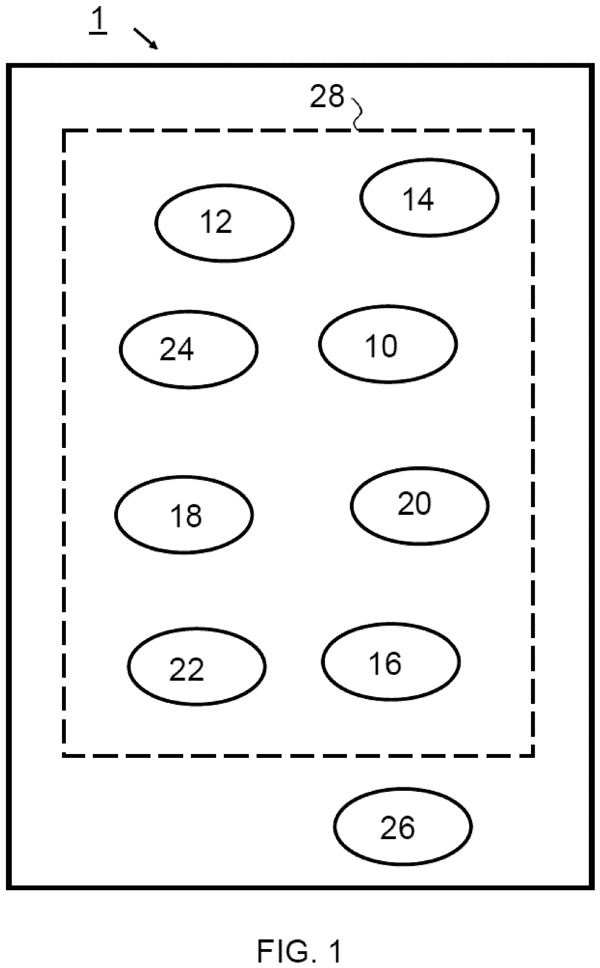

A device 1 for performing a method for determining additional predicted test behavior characteristics of a virtual material as shown in FIG. 1 includes at least one processor 10, at least one user-interface 12, at least one input-module 14, at least one output-module 16, at least one storage 18, an association-module 20, a creation-module 22, a requirement-module 24 and an external-data-source 26. The at least one processor 10, the at least one user-interface 12, the at least one input-module 14, the at least one output-module 16, the at least one storage 18, the association-module 20, the creation-module 22 and the requirement-module 24 can be arranged together in a housing 28 but can also be arranged beside each other without being assembled together in a housing 28. The housing 28 may be fixed to the ground but may alternatively be transportable, so that it can be easily transported from one place to another to be used at different places. All elements of the device 1 are connected to each other in a suitable and well-functioning manner, which is not shown in further detail. The at least one processor 10, the at least one user-interface 12, the at least one input-module 14, the at least one output-module 16, the at least one storage 18, the association-module 20, the creation-module 22, the requirement-module 24 and the external-data-source 26 can be connected with each other physically, for example by a wired connection, which is not shown in further detail, but alternatively some or all of these elements may be connected wireless, which is not shown in further detail.

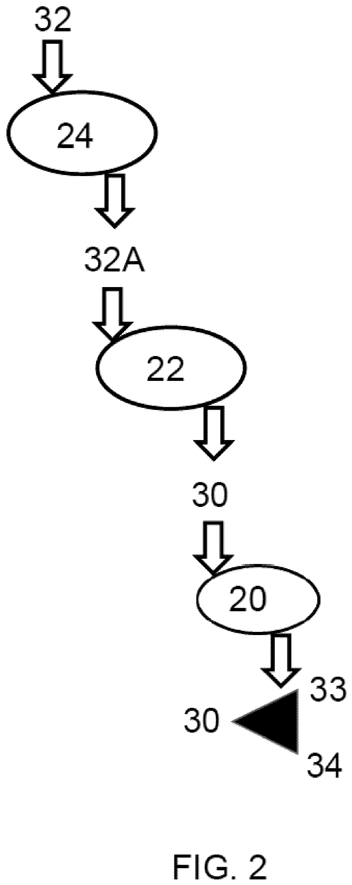

For creating a virtual material 30 predetermined requirements 32 for the virtual material 30 are established as shown in FIG. 2. This can be done for example by a user using the user-interface 12 and feeding some or all predetermined requirements 32 in electronic form into the requirement-module 24. Alternatively or additionally some or all predetermined requirements 32 for the virtual material 30 can be provided electronically from the external-data-source 26, which may be for example an external user-interface and/or an external database. The predetermined requirements 32 are fed appropriately, which is not described and/or shown in further detail, as input in electronic form into the requirement-module 24. The requirement-module 24 receives electronically one or more predetermined requirements 32, which shall be used for creating the virtual material 30, and selects from all these electronic predetermined requirements 32 a set of electronic predetermined requirements 32A. The set of predetermined requirements 32A may include all requirements 32 which were fed into the requirement-module 24 but may also not include all predetermined requirements 32 which were fed into the requirement-module 24. This set of electronic predetermined requirements 32A for the virtual material 30 is transferred electronically in a well-functioning manner, which is not further described or shown in detail, to the creation-module 22. The requirement-module 24 is designed to carry out electronically at least the described functions. The requirement-module has, for example, a storage, a processor, a receiving-unit and an output-unit.

For carrying out the method for determining additional predicted test behaviour characteristics of a virtual material 30 the virtual material 30 is created as shown in FIG. 2. The creation of the virtual material 30 takes place electronically in the creation-module 22. To create the virtual material 30 in the creation-module 22 the set of predetermined requirements 32A for creating a virtual material 30 is fed electronically into the creation-module 22 from the requirement-module 24, which is done in a well-functioning manner but not further described and/or shown in detail. Based on the set of predetermined requirements 32A a virtual material 30 is created electronically in the creation module 22, for example by using electronic data from the at least one storage 18 and/or electronic data from the external-data-source 26. This creation of the virtual material 30 can be done for example by a simulation computer program using artificial intelligence. The creation-module 22 is configured to output a virtual material 30 in electronic form. Furthermore, creation-module 22 is configured to design the virtual material 30 based on the electronic set of predetermined requirements 32A, which the creation module receives from the requirement-module 24. The creation-module 22 is configured to carry out electronically at least the described functions. The creation-module 22 has, for example, a storage, a processor, a receiving-unit and an output-unit to implement at least some of the functions described herein.

Virtual material 30 within the meaning of this disclosure means at least one computer file including a desired formulation satisfying a set of predetermined requirements. This at least one computer file of the virtual material 30 may also include specific properties of the virtual material which can be used alone or in combination for computer simulation purposes. Specific properties of the virtual material 30 can be for example the components, the composition of the components, the aggregate condition of the components, behavior of the components under different temperatures, behavior of the components under different pressures, color of the components and/or toxicity of the components, and/or the like. Virtual material 30 within the meaning of this disclosure may also be understood as a virtual material card of the virtual material 30 including at least one computer file containing specific material properties needed for simulation purposes and being created based on a set of predetermined requirements 32A.

Before starting the method for determining additional predicted test behavior characteristics of a virtual material 30 the virtual material 30 is associated with predicted test behavior characteristics with respect to at least one virtual material test 34 in the association module 20 as shown in FIG. 2. The association module 20 receives electronically the virtual material 30 from the creation-module 22. The association-module 20 associates the virtual material 30 with predicted test behavior characteristics 33 with respect to at least one virtual material test 34. This is done by subjecting the virtual material 30 with a simulation program and/or artificial intelligence in the association-module 20. The association-module has as output in electronic from the virtual material 30 associated with the predicted test behavior characteristics with respect to at least one virtual material test 34. The association-module 20 is configured to carry out electronically at least the described functions. The association-module 20 has, for example, a storage, a processor, a receiving-unit and an output-unit to implement the functions as described above.

Example 1 for performing the method for determining additional predicted test behavior characteristics of a virtual material 30, wherein the virtual material 30 is associated with predicted test behavior characteristics 33 with respect to at least one virtual material test 34, includes at least the following steps S1, S2 and S3 according to FIG: 3 which are all performed by the processor 10. For this purpose, the processor 10 is designed in a well-functioning manner. The processer 10 may, for example, include at least one internal processor, an internal storage, a receiving-unit and an output-unit.

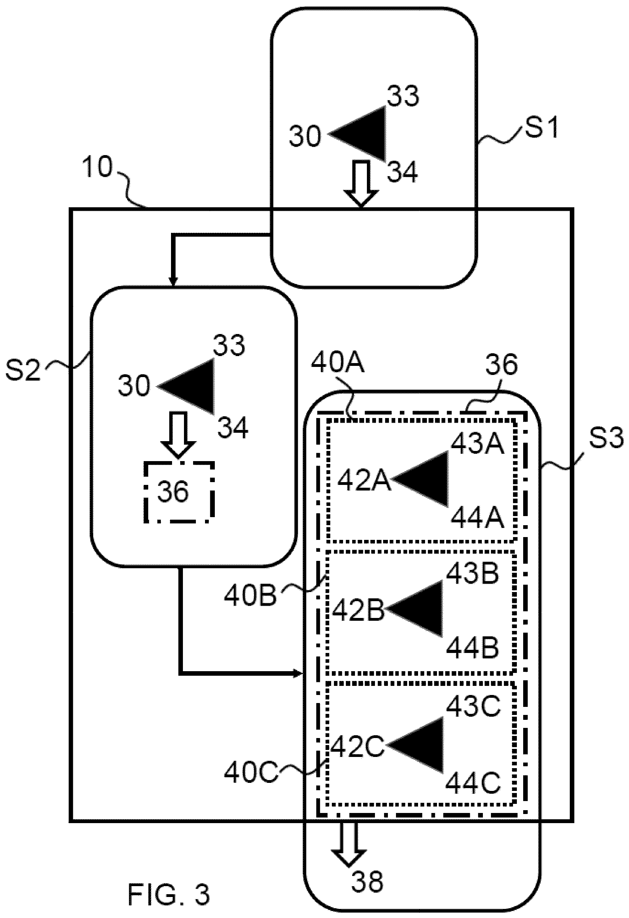

In S1, as shown in FIG. 3, the virtual material 30 and the associated test behavior characteristics 33 of at least one material test 34 are fed electronically from the association-module 20 into the processor 10, which may take place via the receiving-unit from the processor 10.

S1 is followed by S2, as shown in FIG. 3, in which the virtual material 30 and the associated test behavior characteristics 33 of at least one material test 34 are applied to a machine-learning model 36, which may take place in an internal processor of the processor 10.

S2 is followed by S3, as shown in FIG. 3, in which the additional test behavior characteristics 38 of the virtual material 30 are determined in the processor 10 by using the machine-learning model 36. The additional test behavior characteristics 38 may be generated in the processor 10 electronically by the machine-learning model 36 and may be provided as an output from the processor 10 at the end of step S3.

For this purpose, the machine-learning model 36 is adapted (in other words trained) to a plurality of training data sets 40 of which only three training data sets 40A, 40B and 40C are shown for clarity. The plurality of training data sets 40 may include for example more than 100 training data sets or for example even more than 1000 training data sets. The plurality of training data sets 40 may be stored internally in the processor 10, alternatively or additionally in the storage 18 and alternatively or additionally in the external-data-source 26.

Each training data set 40A, 40B and 40C of the plurality of training data sets 40 includes a virtual reference material 42A, 42B and 42C. Each virtual reference material 42A, 42B and 42C is associated with simulated test behavior characteristics 43A, 43B, 43C for a plurality of virtual reference material tests 44A, 44B and 44C. This means that a training data set of the plurality of training data sets 40 includes a virtual reference material and the virtual reference material is associated with simulated test behavior characteristics for a plurality of virtual reference material tests.

For example, the training data set 40A includes a virtual reference material 42A and this virtual reference material 42A is associated with simulated test behavior characteristics 43A for not one but a plurality of virtual material tests 44A referring to the virtual reference material 42A.

If for example, the virtual material 30 is an adhesive, then the training data set 40A includes for example as virtual reference material 42A a combination of binder, modifier and extender. This virtual reference material 42A is associated for example with simulated test behavior characteristics of simulated structural, thermal and rheological tests.

By using electronically the machine-learning model 36 in the processor 10 and carrying out the method for determining additional predicted test characteristics 38 of the virtual material 30 results in generating additional predicted test behavior characteristics 38 of the virtual material 30. The additional predicted test behavior characteristics 38 of the virtual material 30 which are determined in the processor 10 can be provided by the processor 10 at the end of S3 for example to a user via the user-interface 12 and/or can be stored in the storage 18 and/or can be transferred to the external data source 26 for being used, either by a computer program or a user.

The additional predicted test behavior characteristics 38 may include for example simulated structural, thermal and rheological behavior characteristics of the virtual material 30, if the virtual material 30 is for example an adhesive. Providing these additional predicted test behavior characteristics may reduce the number of experiments in reality executed on a real material prepared according to the virtual material 30.

In one embodiment of the present disclosure, each virtual reference material 42A, 42B, 42C is associated with a virtual reference material card, which means that the virtual reference material 42A is associated with a first virtual reference material card, the virtual reference material 42B is associated with a second virtual reference material card and the virtual reference material 42C is associated with a third virtual reference material card. The machine-learning model 36 performs for example a Design Of Experiments (DOE) for some or all parameters of each virtual reference material card. The Design Of Experiments (DOE) generates for each virtual material 42A, 42B, 42C associated simulated test behavior characteristics for a plurality of virtual material tests 44A, 44B, 44C and thus generates training data sets 40A, 40B, 40C. Alternatively the DOE for some or all parameters of each virtual reference material card may be performed externally and the results of the DOE may be fed into the external-data-source 26 to be transferred in a non-shown manner to the machine-learning model 36. The machine-learning model 36 connects the training data sets 40A, 40B, 40C with the virtual material 30 and the association of the virtual material 30 to predicted test behavior characteristics 33 of at least one material test 34 for example by using an artificial algorithm and provides in this way additional predicted test behavior characteristics for the virtual material 30. This means that by using the machine-learning model 36 additional predicted test behavior characteristics 38 of the virtual material 30, which is associated with predicted test behavior characteristics of at least one material test, can be determined.

Design Of Experiments or DOE as used in this disclosure means a branch of applied statistics that deals with planning, conducting, analyzing, and interpreting controlled test simulations to evaluate parameters of at least one material card of a virtual material and/or of at least one material card of a virtual reference material and/or of at least one material card of a further virtual reference material.

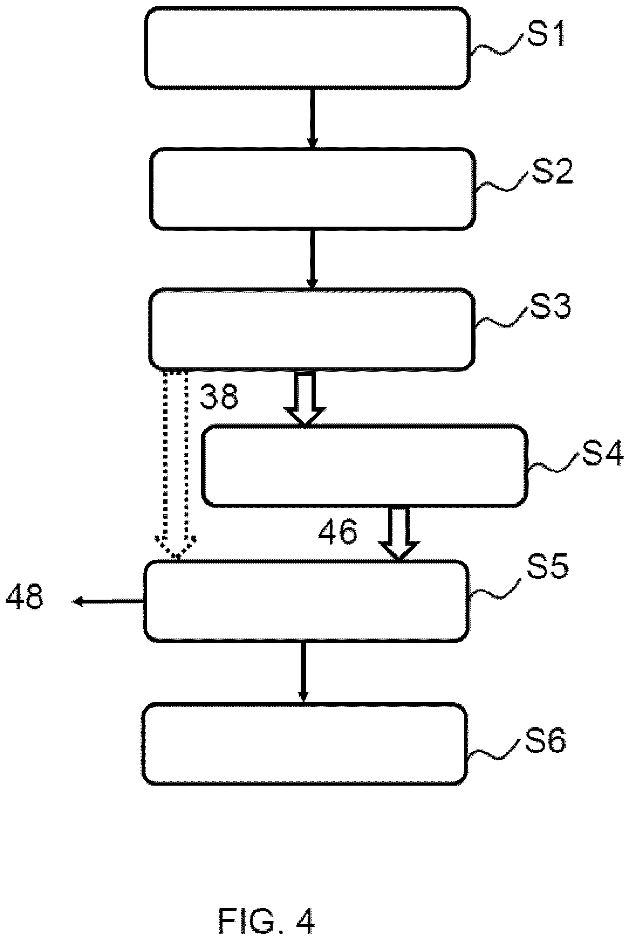

In Example 2, the subject matter of Example 1 can optionally include, as shown in FIG. 4, that S3 is followed by S4, in which a virtual material card 46 is determined electronically for the virtual material 30 including the additonal predicted test behavior characteristics 38. The virtual material card 46 can be distributed electronically to be used, for example to the user-interface 12 and/or the storage 18 and/or the external- data-source 26. The determination of the virtual material card 46 can take place in the processor 10 or alternatively in a further not shown module of the device 1. The virtual material card 46 can be used for example to simulate and test the behavior of the virtual material 30 virtually.

In Example 3, the subject matter of any one of Examples 1 or 2 can optionally include that in S5, as shown in FIG. 4 a real material 48 is prepared corresponding to the virtual material 30. S5 can, for example, be carried out after S4, but can alternatively also be carried out directly after S3. S5 takes place in the physical world and thus not within the device 1. For this reason, the electronic data including the virtual material 30 are transferred to a user. This can be done via the user-interface 12 and/or via a user interface of the the external-data-source 26.

In Example 4, the subject matter of any one of Examples 1 to 3 can optionally include that after S5, S6, as shown in FIG. 4 is carried out, in which the virtual material test 34 is performed in the physical world on the prepared real material 48 and not electronically. Alternatively or additonally a (real physical) material test can be performed in the physical world on the prepared real (physical) material 48 which corresponds to at least one virtual reference material test of the plurality of virtual reference material tests 44A, 44B, 44C of the virtual reference materials 42A, 42B, 42C. S6 takes place in the physical world and thus not within the device 1.

In Example 5, the subject matter of any one of Examples 1 to 4 can optionally include that the machine-learning model 36 of S3 includes a neural network.

In Example 6, the subject matter of Example 5 can optionally include that the machine-learning model 36 of S3 includes a long short-term memory neural network.

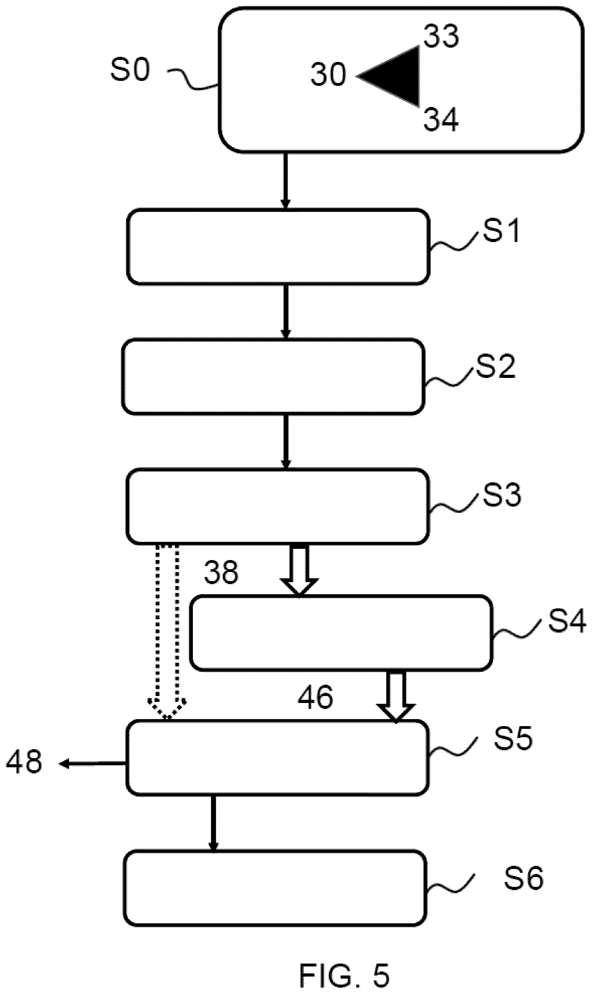

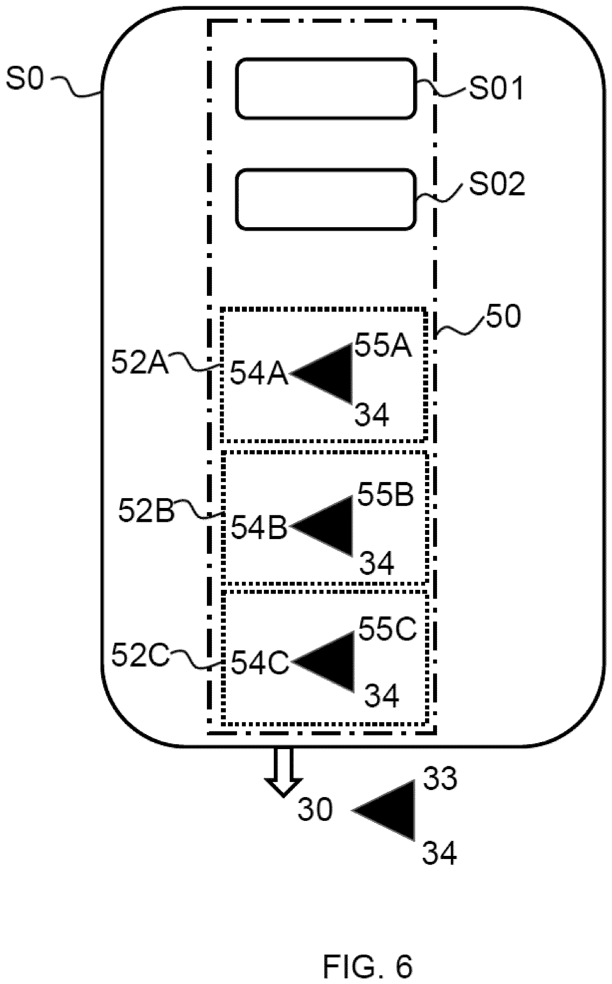

In Example 7, the subject matter of any one of Examples 1 to 6 can optionally include S0, as shown in FIG. 5 and FIG. 6, in which the virtual material 30 is determined using a further machine-learning model 50. The further machine learning model 50 can be the same as the machine-learning model 36 but can also be suitably different from the machine-learning model 36. The further machine learning model 50 can be implemented in an internal processor of the processor 10. In this case the virtual material 30 is determined in the processor 10 by using the further machine-learning model 50 and not in the creation module 22.

The association of the virtual material 30, which was determined by using the further machine-learning model 50 in the processor 10, with predicted test behavior characteristics 33 with respect to at least one virtual material test 34 then takes place, for example, in the association module 20. To do this, the processor 10 feeds the determined virtual material 30 into the the association-module 20. After associating the virtual material 30 determined by using the further machine-learning model 50 with predicted test behavior characteristics 33 with respect to at least one virtual material test 34, S1 of the method is performed, in which the virtual material 30 and the associated predicted test behavior characteristics 33 of at least one material test 34 are fed electronically from the association-module 20 into the processor 10. Alternatively the further machine-learning model 50 may be implemented by a processor of the creation-module 22. In this case, for example, the virtual material 30 is determined using the further machine-learning model 50 in the creation module 22 and may then be fed into the association module 20 for being associated with predicted test behavior characteristics 33 of at least one material test 34 and then S1 of the method according to the disclosure may be performed.

The further machine-learning model 50 is adapted to a plurality of further training data sets 52 of which only three further training data sets 52A, 52B and 52C are shown for clarity. The plurality of further training data sets 52 may include for example more than 100 further training data sets or alternatively for example more than 1000 further training data sets. The plurality of further training data sets 52 may be stored internally in the processor 10 and/or in the storage 18 and/or in the external-data-source 28 and/or in a storage of the creation-module 22.

Each further training data set 52A, 52B and 52C of the plurality of further training data sets 52 includes a further virtual reference material 54A, 54B and 54C. Each further virtual reference material 54A, 54B and 54C is associated with simulated test behavior characteristics 55A, 55B, 55C of the at least one virtual material test 34 referring to the respective further virtual reference material 54A, 54B, 54C.

For example, if the virtual material 30 is an adhesive, the further virtual reference materials 54A, 54B, 54C may include three different combinations of binders, modifiers and/or extenders. In this case, the associated simulated test behavior characteristics 55A, 55B, 55C may include the results of focused testing with respect to the at least one material test 34. The at least one material test 34 can be for example a lapshear test for structural simulated test behavior characteristics, a thermal conductivity for thermal simulated test behavior characteristics or a parallel plate test for rheological simulated test behavior characteristics. All the information for the Design of Experiments is provided for example in the storage 18 and used for generating the simulated test behavior characteristics with respect to at least one virtual material test 34.

If for example, the virtual material test 34 is a lapshear test, then the simulated test behavior characteristics 55A, 55B, 55C include structural simulated test behavior characteristics for three different combinations of binders, modifiers and/or extenders being the three further virtual reference materials 54A, 54B and 54C in this example.

Based on the lapshear test as the at least one virtual material test 34 and the structural simulated test behavior characteristics for three different combinations of binders, modifiers and/or extenders being the three further virtual reference materials 54A, 54B and 54C in this example an adhesive as virtual material 30 is created in step S0 using the further machine-learning model 50.

In Example 8, the subject matter of Example 7 can optionally include that the further machine-learning model 50 includes a neural network.

In Example 9, the subject matter of Example 8 can optionally include that the further machine-learning model 50 includes a long short-term neural network.

In Example 10, the subject matter of any one of Examples 7 to 9 can optionally include that desired material characteristics are applied to the further machine-learning model 50. This can be done for example in S01 being part of S0 as shown in FIG. 6. Applying desired material characteristics to the further machine-learning model 50 can take place in a well-functioning manner either in the processor 10 or in the requirement-module 24. In case that applying desired material characteristics to the further machine-learning model 50 takes place in the requirement module 24, the machine-learning model 50 is as well part of the requirement-module 24. The desired material characteristics can be fed electronically into the device 1 via the user-interface 12 and/or may be received from the external-data-source 26 and/or may be received from the storage 18. The desired material characteristics can be the same as the set of predetermined requirements 32A but can alternatively include more or less predetermined requirements 32 for the virtual material 30 to be determined by the further machine-learning model 50. The desired material characteristics can also be based on requirements defined by a user and fed into the device 1 via the user-interface 12.

In Example 11, the subject matter of Example 10 can optionally include that the desired material characteristics include desired physical properties of the virtual material 30, e.g. at least one of a desired size of the virtual material 30, a desired ratio of material components of the virtual material 30, and/or a desired treatment required for manufacturing a material 48 corresponding to the virtual material 30.

In Example 12, the subject matter of any one of Examples 7 to 11 can optionally include, as shown in FIG. 6, S02, in which determining the virtual material 30 further includes determining predicted test behaviour characteristics 33 with respect to the at least one virtual material test 34 for the virtual material 30 using the further machine-learning model 50. S02 can be part of S0 as shown in FIG: 6 and may be performed after S01.

In Example 13, the subject matter of any one of Examples 1 to 12 can optionally include that the at least one virtual material test 34 is selected from a group of virtual material tests consisting of:
- a structural material test, e.g. one or more of a dogbone test, a lap shear test, a thick lap shear test, a tapered-double-cantilever beam (tdcb) test, an end-notched flexure (enf) test, a butt joint test, and a hollow cylinder test;
- a thermal material test e.g. thermal conductivity test, specific heat capacity test; and
- a rheological test, e.g. a parallel plate compression test and capillary tube test.

In Example 14, the subject matter of any one of Examples 1 to 13 can optionally include that the at least one virtual reference material test of the plurality of virtual reference material tests is selected from a group of virtual reference material tests consisting of at least one of:
- a structural material test, e.g. one or more of a dogbone test, a lap shear test, a thick lap shear test, a tapered-double-cantilever beam (tdcb) test, an end-notched flexure (enf) test, a butt joint test, and a hollow cylinder test;
- a thermal material test e.g. thermal conductivity test, specific heat capacity test; and
- a rheological test, e.g. a parallel plate compression test and capillary tube test.

In Example 15, the subject matter of any one of Examples 1 to 14 can optionally include that the virtual material 30 is associated with predicted test behaviour characteristics 33 with respect to exactly one virtual material test 34.

In one embodiment of the present disclosure, the desired material characteristics according to Example 10 are the same as the set of predetermined requirements 34 and include to develop a thermal conductive adhesive (TCA) with 5MPA lapshear strength, 2000MPA E-Modulus and 2W/mK thermal conductivity. This set of predetermined requirements 32A is fed electronically into the device 1 via the user-interface 12 and applied to the further machine-learning model 50, which in this case is implemented in the processor 10 of the device 1, in step S01 being part of step S0 as shown in FIG. 6.

The virtual TCA as virtual material 30 can then be determined in S0 as shown in FIG. 6 using the further machine-learning model 50, implemented in this case in the processor 10, according to Example 7. The further machine-learning model 50 in this example uses the desired material characteristics, which are in this example the same as the set of predetermined requirements 32A, applied to the further machine-learning model 50 according to Example 10. This means that the virtual TCA is determined by the machine-learning model 50 using 5MPA lapshear strength, 2000MPA E-Modulus and 2W/mK thermal conductivity as desired material requirements. The machine-learning model 50 may use an artificial algorithm when determining the virtual TCA using 5MPA lapshear strength, 2000MPA E-Modulus and 2W/mK thermal conductivity as desired material requirements.

As example for a further training data set 52, the further training data set 52A includes a first formulation of TCA as further virtual reference material 54A and a lap shear test as the exactly one virtual material test 34 and associated test behavior characteristics, which are determined using DOE for a virtual reference material card of the further virtual reference material 54A.

The result of S0 is virtual TCA as virtual material 30, which is associated with predicted test behavior characteristics 33 with respect to the lap shear test as the exactly one virtual material test 34. The exactly one virtual material test may be called focused test and the predicted test behavior characteristics may be called focused test behavior.

The result of S0 is used in this example to perform S1, S2 and S3 as described above. The additional predicted test behavior characteristics 38 are in this example cohesive zone modelling (CZM) behavior characteristics for the virtual TCA as virtual material 30.

In Example 16, the subject matter of any one of Examples 1 to 15 can optionally include that the simulated test behavior characteristics are determined using one or more simulation programs.

In Example 17, the subject matter of any one of Examples 1 to 16 can optionally include that the simulated test behaviour characteristics are determined using a finite element-based simulatin method.

In Example 18, the subject matter of any one of Examples 1 to 17 can optionally include that the simulated test behaviour characteristics are determined in a predefined simulation parameter range.

In Example 19, the subject matter of any one of Examples 1 to 18 can optionally include that the simulated test behaviour characteristics are determined in a predefined simulation parameter range by performing the simulation at least at a first input parameter, e.g. a minimum parameter of the simulation parameter range, and at a second input parameter, preferably a maximum parameter of the simulation parameter range.

In Example 20, the subject matter of Example 19 can optionally include that the simulated test behaviour characteristics are determined in a predefined simulation parameter range by performing the simulation at a plurality of input parameters within the simulation parameter range, starting from the first input parameter to the second input parameter.

Using according to Example 18, Example 19 and Example 20 a predefined simulation parameter range for the simulated test behavior characteristics provides for varying the extremes of the parameters of the virtual material 30 and/or the virtual material card 46 made for the virtual material 30.

In Example 21, the subject matter of any one of Examples 1 to 20 can optionally include that the virtual material 30 includes at least one of the following materials: a binder, a modifier; and/or an extender.

In Example 22, the subject matter of any one of Examples 1 to 21 can optionally include that the virtual material 30 is an adhesive formulation. In general, a formulation of the adhesive may consist of a combination of different materials such as binders, modifiers and/or extenders.

In Example 23, a computer readable storage medium storing instructions which, when executed by a processor, implements a method or subject matter of any one of Examples 1 to 22. The computer readable storage medium can be the storage 18 of the device 1. The processor can be the processor 10 of the device 1.

In Example 24 a device for determining additional predicted test behavior characteristics 38 of a virtual material 30, including a processor 10 configured to implement a method of any one of Examples 1 to 22 can be the device 1.

This disclosure helps to identify the formulation of the material for the given requirement, as well as it provides the characteristics of the material in terms of mechanical, thermal and rheological behavior. This disclosure aims to address formulation and material behavior and the bridge between them.

The disclosure provides the development of a predictive machine algorithm that can relate the effect of different components in adhesive formulation to the physical behavior of the material using focused test. Therefore, this disclosure relates the effect of different components in adhesive formulation to focused testing, and then relates the focused testing to physical behavior of the material such as thermal, mechanical and rheological characteristics.

This disclosure provides among others a predictive machine learning method to identify the structural, thermal and rheological behavior of an adhesive using focused test and the database of simulation results. This disclosure also addresses the development of a predictive machine algorithm that can relate the effect of different components in adhesive formulation to the physical behavior of the material using focused test. Therefore, this disclosure can relate the effect of different components in adhesive formulation to focused testing, and then relating the focused testing to physical behavior of the material such thermal, mechanical and rheological characteristics.

The disclosure provides acceleration of adhesive formulation development for the given requirements using a machine-learning model.

The disclosure further provides acceleration of the creation of virtual material cards or material data cards by reducing the experimental efforts

In addition, the disclosure provides the prediction of adhesive material properties with regard to mechanical, thermal and rheological properties.

Also, the disclosure provides for more effective, faster and wider range of material selection options for given requirements.

While the invention has been particularly shown and described with reference to specific embodiments, it should be understood by those skilled in the art that various changes in form and detail may be made therein without departing from the spirit and scope of the invention as defined by the appended claims. The scope of the invention is thus indicated by the appended claims and all changes which come within the meaning and range of equivalency of the claims are therefore intended to be embraced.

### List of reference signs

- 1: device
- 10: processor
- 12: user-interface
- 14: input-module
- 16: output-module
- 18: storage
- 20: association-module
- 22: creation-module
- 24: requirement-module
- 26: external-data-source
- 30: virtual material
- 32: predetermined requirements
- 32A: set of predetermined requirements
- 33: predicted test behavior characteristics
- 34: one material test
- 36: machine-learning model
- 38: additional predicted test behavior characteristics
- 40: plurality of data sets
- 40A, 40B, 40C: training data set
- 42A,42B, 42C: virtual reference material
- 43A, 43B, 43C: associated simulated test behavior characteristics
- 44A, 44B, 44C: virtual material test
- 46: virtual material card
- 48: material
- 50: further machine-learning model
- 52: plurality of further training data sets
- 52A, 52B, 52C: further training data set
- 54A, 54B, 54C: further virtual reference material
- 55A, 55B, 55C: associated simulated test behavior characteristics
- S1, S2, S3: step
- S4, S5, S6: step
- S0: step
- S01, S02: step

## Claims

1. A method for determining additional predicted test behavior characteristics of a virtual material, wherein the virtual material is associated with predicted test behavior characteristics with respect to at least one virtual material test, the method performed by a processor, the method comprising:
applying the virtual material and the associated predicted test behavior characteristics with respect to at least one virtual material test to a machine-learning model, wherein the machine-learning model is adapted to a plurality of training data sets, each training data set of the plurality of training data sets comprising
a virtual reference material and
associated simulated test behavior characteristics for a plurality of virtual reference material tests referring to the respective virtual reference material,
wherein the virtual reference materials are different from the virtual material,
wherein at least some virtual reference material tests are different from the at least one virtual material test;
determining the additional predicted test behavior characteristics of the virtual material using the machine-learning model.

2. The method of claim 1, further comprising:
determining a virtual material card for the virtual material including the additional test behavior characteristics of the material.

3. The method of any one of claims 1 or 2, further comprising:
preparing a material corresponding to the virtual material;
preferably the method further comprising performing the virtual material test in reality and/or at least one virtual reference material test of the plurality of virtual reference material tests in reality on the prepared material.

4. The method of any one of claims 1 to 3,
wherein the machine-learning model comprises a neural network;
preferably wherein the machine-learning model comprises a long short-term memory neural network.

5. **The** method of any one of claims 1 to 4, further comprising:
determining the virtual material using a further machine-learning model,
wherein the further machine-learning model is adapted to a plurality of further training data sets, each further training data set of the plurality of data sets comprising
a further virtual reference material and
associated test behavior characteristics for the at least one virtual material test referring to the respective further virtual reference material;
preferably wherein the further machine-learning model comprises a neural network;
further preferably wherein the further machine-learning model comprises a long short-term memory neural network.

6. The method of claim 5, further comprising:
applying desired material characteristics to the further machine-learning model;
preferably wherein the desired material characteristics comprise desired physical properties of the virtual material, preferably at least one of a desired size of the virtual material, a desired ratio of material components of the virtual material, and/or a desired treatment required for manufacturing a material corresponding to the virtual material.

7. The method of any one of claims 5 or 6,
wherein determining the virtual material further comprises determining predicted test behavior characteristics with respect to at least one virtual material test for the virtual material using the further machine-learning model.

8. The method of any one of claims 1 to 7,
wherein the at least one virtual material test is selected from a group of virtual material tests consisting of:
a structural material test, preferably one or more of a dogbone test, a lap shear test, a thick lap shear test, a tapered-double-cantilever beam (tdcb) test, an end-notched flexure (enf) test, a butt joint test, and a hollow cylinder test;
a thermal conductivity test; and
a rheological test, preferably a parallel plate compression test.

9. The method of any one of claims 1 to 8,
wherein at least one virtual reference material test of the plurality of virtual reference material tests is selected from a group of virtual reference material tests consisting of at least one of:
a structural material test, preferably one or more of a dogbone test, a lap shear test, a thick lap shear test, a tapered-double-cantilever beam (tdcb) test, an end-notched flexure (enf) test, a butt joint test, and a hollow cylinder test;
a thermal conductivity test, preferably; and
a rheological test, preferably a parallel plate compression test.

10. The method of any one of claims 1 to 9,
wherein the virtual material is associated with predicted test behavior characteristics with respect to exactly one virtual material test; and/or wherein the simulated virtual material test behavior characteristics are determined using one or more simulation programs; and/or wherein the simulated virtual material test behavior characteristics are determined using a finite element-based simulation method.

11. The method of any one of claims 1 to 10,
wherein the simulated test behavior characteristics are determined in a predefined simulation parameter range;
preferably wherein the simulated test behavior characteristics are determined in a predefined simulation parameter range by performing the simulation at least at a first input parameter, preferably a minimum parameter of the simulation parameter range, and at a second input parameter, preferably a maximum parameter of the simulation parameter range;
further preferably wherein the simulated test behavior characteristics are determined in a predefined simulation parameter range by performing the simulation at a plurality of input parameters within the simulation parameter range, starting from the first input parameter to the second input parameter.

12. The method of any one of claims 1 to 11,
wherein the virtual material comprises at least one of the following materials:
a binder;
a modifier; and/or
an extender.

13. The method of any one of claims 1 to 12,
wherein the virtual material is an adhesive formulation.

14. A computer readable storage medium storing instructions which, when executed by a processor, implements a method of any one of claims 1 to 13.

15. A device for determining additional predicted test behavior characteristics of a virtual material, comprising a processor configured to implement a method of any one of claims 1 to 13.
